# EUROPEAN PATENT APPLICATION

(11) **EP 4 230 725 A1**
(43) Date of publication of application: **23.08.2023**
(21) Application number: 21895081.4
(22) Date of filing: 17.11.2021
(51) Int. Cl.: C12N 5/071, C12M 3/00

(54) **ESOPHAGUS EXTRACELLULAR MATRIX-DERIVED SCAFFOLD FOR CULTURING AND TRANSPLANTING ESOPHAGEAL ORGANOID, AND METHOD FOR PRODUCING SAME**

(30) Priority: 17.11.2020 KR 20200153717; 16.11.2021 KR 20210157355
(71) Applicant: Cellartgen Inc., Seoul 03722 (KR)
(72) Inventor: CHO, Seung Woo, Seoul 03722 (KR); PARK, Se Won, Seoul 03722 (KR); JEON, Eun Je, Seoul 03722 (KR)
(74) Representative: Manitz Finsterwald Patent- und Rechtsanwaltspartnerschaft mbB
(86) International application number: PCT/KR2021/016825
(87) International publication number: WO 2022/108313

(57) **Abstract**

The present disclosure relates to a scaffold for culture and transplantation of an esophageal organoid by using a decellularized esophageal tissue (esophagus extracellular matrix; EEM).

## Description

### TECHNICAL FIELD

The present disclosure relates to an esophagus extracellular matrix-derived scaffold for culture and transplantation of an esophageal organoid and a method of preparing the same.

### BACKGROUND

An organoid can mimic the structure and function of a specific organ in a three-dimensional environment and thus is regarded as a promising in vitro model that can be applied to various biomedical fields, such as developmental research, disease modeling, new drug development, drug screening, and cell therapy product. In general, commercialized Matrigel has been used as an organoid culture scaffold. However, Matrigel is a component derived from mouse sarcoma cells, and has the risk of causing infection with animal pathogens and serving as an immunogen. Also, Matrigel cannot provide a tissue-specific microenvironment, and is highly expensive. Therefore, an organoid culture system based on Matrigel has practical limits. While there have been some studies on the development of various hydrogel scaffolds to replace Matrigel, no material has been reported that can replace Matrigel.

Decellularization is the process of removing cells from a tissue to reduce the risk of immune responses and preserving various extracellular components to implement a tissue-specific microenvironment and thus has attracted a lot of attention for use in fabricating a functional scaffold in the tissue engineering field. However, there has been no study to evaluate the possibility of replacing Matrigel with an extracellular matrix by comparing and analyzing equivalence between an esophageal organoid cultured using an esophageal tissue-specific extracellular hydrogel fabricated by decellularization and an esophageal organoid cultured in Matrigel.

In the present disclosure, it was verified that when an extracellular hydrogel scaffold derived from a decellularized esophageal tissue is applied to culture of an esophageal organoid, an esophageal organoid can be formed and maintained at a similar level to that in Matrigel. In particular, the expression of esophageal tissue-specific markers was greatly improved in the esophageal organoid cultured in the hydrogel derived from the decellularized esophageal tissue compared to the esophageal organoid cultured in Matrigel. That is, it was verified that the developed decellularized scaffold can provide an esophageal tissue-specific microenvironment optimized for an esophageal organoid. Therefore, the decellularized esophageal tissue-derived hydrogel scaffold can be developed as a novel esophageal organoid culture platform that can overcome the limitations of conventional Matrigel-based organoids, based on the advantages of decellularization that enables mass production at low cost.

### DISCLOSURE OF THE INVENTION

### PROBLEMS TO BE SOLVED BY THE INVENTION

The present disclosure is conceived to obtain a large amount of decellularized tissue through a chemical treatment of a porcine esophageal tissue, prepare a hydrogel scaffold based on the decellularized tissue and apply it to esophageal organoid culture.

However, the problems to be solved by the present disclosure are not limited to the above-described problems. Although not described herein, other problems to be solved by the present disclosure can be clearly understood by a person with ordinary skill in the art from the following descriptions.

### MEANS FOR SOLVING THE PROBLEMS

An aspect of the present disclosure provides a scaffold for culture and transplantation of an esophageal organoid, containing an esophagus extracellular matrix (EEM).

In an embodiment of the present disclosure, the EEM may be decellularized by treating an esophageal tissue with trypsin/EDTA, Triton X-100, and sodium deoxycholate.

In an embodiment of the present disclosure, a concentration of the EEM in the scaffold may be from 0.5 mg/ml to 10 mg/ml.

Another aspect of the present disclosure provides a method of preparing a scaffold for culture and transplantation of an esophageal organoid, including: a process (1) of crushing an isolated esophageal tissue; and a process (2) of treating the crushed esophageal tissue with trypsin/EDTA, Triton X-100, and sodium deoxycholate for decellularization to prepare a decellularized EEM.

In an embodiment of the present disclosure, the method may further include, after the process (2), a process (3) of lyophilizing the decellularized EEM to prepare a lyophilized EEM.

In an embodiment of the present disclosure, the method may further include, after the process (3), a process (4) of forming a scaffold for culture and transplantation of an esophageal organoid in the form of a hydrogel with the lyophilized EEM.

In an embodiment of the present disclosure, in the process (4), the lyophilized EEM may be dissolved in a pepsin solution and a pH of the solution may be adjusted to form the hydrogel.

Yet another aspect of the present disclosure provides a method of culturing an esophageal organoid in the above-described scaffold or a scaffold prepared by the above-described preparation method.

### EFFECTS OF THE INVENTION

A decellularized esophageal tissue-derived scaffold developed in the present disclosure can be applied as a novel platform that can overcome the limitations of Matrigel, which is a conventional organoid culture scaffold, and can fabricate an esophageal organoid equivalent to Matrigel. Therefore, it is expected to construct an in vitro esophagus model and to be applied to various biomedical fields, such as disease modeling, new drug screening, and transplantation therapy for tissue regeneration, using an esophageal organoid. Accordingly, it is expected to make a signification contribution to basic studies and medical industries.

The prevalence of digestive diseases is rapidly increasing in Korea as well due to various factors such as westernization of dietary patterns and aging. Not only in Korea but also worldwide, dietary patterns, such as increased uptake of instant foods and irregular eating, in modern countries cause an increase in prevalence of digestive diseases. Among the digestive diseases, various esophagus-related diseases can become chronic once established and are difficult to treat. Also, the esophagus-related diseases bring lifelong pain to patients and thus significantly degrades the quality of life of the patients, and may worsen to esophageal stricture or cancer. As such, the esophagus-related diseases are intractable diseases which are rapidly increasing in modern societies. In view of the need for disease modeling research to implement the esophagus-related diseases (gastroesophageal reflux disease, Barrett's esophagus, oesophageal adenocarcinoma, etc.), which greatly affect the quality of life of modern people, in vitro and to investigate the mechanisms thereof and the need for new drug development, the decellularized esophageal tissue-derived matrix fabricated according to the present disclosure can serve as an esophageal organoid production platform and is expected to create high added value.

In particular, the decellularized esophageal tissue-derived matrix can be mass produced from animal esophageal tissues (e.g., pigs) through a relatively simple chemical treatment and thus shows promise of commercialization. Also, it has a high potential for cost advantages over conventional Matrigel and thus can create huge economic profits. It can be applied as a material for culture of an esophageal organoid, and it can also be applied as a material for transplantation of an esophageal organoid to enhance in vivo engraftment and maintenance of the esophageal organoid when the esophageal organoid is used for regenerative medicine. That is, the decellularized esophageal tissue-derived matrix can have an extended range of application.

### BRIEF DESCRIPTION OF THE DRAWINGS

**FIG. 1a** shows the fabrication of a decellularized esophageal tissue-derived scaffold and the analysis of a hydrogel microstructure.
**FIG. 1b** shows the fabrication of a decellularized esophageal tissue-derived scaffold and the analysis of a hydrogel microstructure.
**FIG. 1c** shows the fabrication of a decellularized esophageal tissue-derived scaffold and the analysis of a hydrogel microstructure.
**FIG. 2a** shows the analysis of components of the decellularized esophageal tissue-derived scaffold.
**FIG. 2b** shows the analysis of components of the decellularized esophageal tissue-derived scaffold.
**FIG.** 3 shows the analysis of mechanical properties of the decellularized esophageal tissue-derived hydrogel scaffold depending on concentration.
**FIG. 4a** shows the proteomics of the decellularized esophageal tissue-derived scaffold.
**FIG. 4b** shows the proteomics of the decellularized esophageal tissue-derived scaffold.
**FIG. 4c** shows the proteomics of the decellularized esophageal tissue-derived scaffold.
**FIG. 5a** shows the result of comparing protein components of the decellularized esophageal tissue-derived scaffold with protein components of Matrigel.
**FIG. 5b** shows the result of comparing protein components of the decellularized esophageal tissue-derived scaffold with protein components of Matrigel.
**FIG. 5c** shows the result of comparing protein components of the decellularized esophageal tissue-derived scaffold with protein components of Matrigel.
**FIG. 5d** shows the result of comparing protein components of the decellularized esophageal tissue-derived scaffold with protein components of Matrigel.
**FIG. 6a** shows the result of checking the similarity between batches of the decellularized esophageal tissue-derived scaffold.
**FIG. 6b** shows the result of checking the similarity between batches of the decellularized esophageal tissue-derived scaffold.
**FIG. 7a** shows the determination of an optimal concentration of the decellularized esophageal tissue-derived hydrogel scaffold for esophageal organoid culture.
**FIG. 7b** shows the determination of an optimal concentration of the decellularized esophageal tissue-derived hydrogel scaffold for esophageal organoid culture.
**FIG. 7c** shows the determination of an optimal concentration of the decellularized esophageal tissue-derived hydrogel scaffold for esophageal organoid culture.
**FIG. 8** shows the growth pattern of an esophageal organoid cultured in the decellularized esophageal tissue-derived hydrogel scaffold.
**FIG. 9a** shows the analysis of gene and protein expression of the esophageal organoid cultured in the decellularized esophageal tissue-derived hydrogel scaffold.
**FIG. 9b** shows the analysis of gene and protein expression of the esophageal organoid cultured in the decellularized esophageal tissue-derived hydrogel scaffold.
**FIG. 10a** verifies the tissue-specific effect of the decellularized esophageal tissue-derived scaffold for esophageal organoid culture.
**FIG. 10b** verifies the tissue-specific effect of the decellularized esophageal tissue-derived scaffold for esophageal organoid culture.
**FIG. 10c** verifies the tissue-specific effect of the decellularized esophageal tissue-derived scaffold for esophageal organoid culture.
**FIG. 11a** verifies the possibility of long-term cold storage of the decellularized esophageal tissue-derived hydrogel scaffold.
**FIG. 11b** verifies the possibility of long-term cold storage of the decellularized esophageal tissue-derived hydrogel scaffold.
**FIG. 11c** verifies the possibility of long-term cold storage of the decellularized esophageal tissue-derived hydrogel scaffold.
**FIG. 12a** verifies the possibility of long-term cryopreservation of the decellularized esophageal tissue-derived hydrogel scaffold.
**FIG. 12b** verifies the possibility of long-term cryopreservation of the decellularized esophageal tissue-derived hydrogel scaffold.
**FIG. 12c** verifies the possibility of long-term cryopreservation of the decellularized esophageal tissue-derived hydrogel scaffold.
**FIG. 13a** verifies the long-term culture of an esophageal organoid in the decellularized esophageal tissue-derived hydrogel scaffold.
**FIG. 13b** verifies the long-term culture of an esophageal organoid in the decellularized esophageal tissue-derived hydrogel scaffold.
**FIG. 14a** shows the result of verifying in vivo transplantation and regeneration effects of an esophageal organoid using the decellularized esophageal tissue-derived hydrogel.
**FIG. 14b** shows the result of verifying in vivo transplantation and regeneration effects of an esophageal organoid using the decellularized esophageal tissue-derived hydrogel.
**FIG. 15c** shows the result of verifying in vivo transplantation and regeneration effects of an esophageal organoid using the decellularized esophageal tissue-derived hydrogel.
**FIG. 15d** shows the result of verifying in vivo transplantation and regeneration effects of an esophageal organoid using the decellularized esophageal tissue-derived hydrogel.
**FIG. 16e** shows the result of verifying in vivo transplantation and regeneration effects of an esophageal organoid using the decellularized esophageal tissue-derived hydrogel.
**FIG. 16f** shows the result of verifying in vivo transplantation and regeneration effects of an esophageal organoid using the decellularized esophageal tissue-derived hydrogel.
**FIG. 16g** shows the result of verifying in vivo transplantation and regeneration effects of an esophageal organoid using the decellularized esophageal tissue-derived hydrogel.
**FIG. 16h** shows the result of verifying in vivo transplantation and regeneration effects of an esophageal organoid using the decellularized esophageal tissue-derived hydrogel.

### BEST MODE FOR CARRYING OUT THE INVENTION

Hereafter, the present disclosure will be described in detail with reference to the accompanying drawings. However, it is to be noted that the present disclosure is not limited to examples described herein but can be embodied in various other ways. It is to be understood that the term "comprises or includes" and/or "comprising or including" used in the document means that one or more other components, steps, operation and/or existence or addition of elements are not excluded in addition to the described components, steps, operation and/or elements unless context dictates otherwise.

Unless otherwise indicated, the practice of the disclosure involves conventional techniques commonly used in molecular biology, microbiology, protein purification, protein engineering, protein and DNA sequencing, and recombinant DNA fields, which are within the skill of the art. Such techniques are known to a person with ordinary skill in the art and are described in numerous standard texts and reference works.

Unless otherwise defined herein, all technical and scientific terms used herein have the same meaning as commonly understood by a person with ordinary skill in the art to which this disclosure belongs.

Various scientific dictionaries that include the terms included herein are well known and available to those in the art. Although any methods and materials similar or equivalent to those described herein find use in the practice or testing of the disclosure, some preferred methods and materials are described. It is to be understood that this disclosure is not limited to the particular methodology, protocols, and reagents described, as these may vary, depending upon the context in which they are used by a person with ordinary skill in the art. Hereinafter, the present disclosure will be described in more detail.

An aspect of the present disclosure provides a scaffold for culture and transplantation of an esophageal organoid, containing an esophagus extracellular matrix (EEM).

The term "extracellular matrix" refers to a natural scaffold for cell growth that is prepared by decellularization of tissue found in mammals and multicellular organisms. The extracellular matrix can be further processed through dialysis or crosslinking.

The extracellular matrix may be a mixture of structural or non-structural biomolecules including, but not limited to, collagens, elastins, laminins, glycosaminoglycans, proteoglycans, antimicrobials, chemoattractants, cytokines and growth factors.

In mammals, the extracellular matrix may contain about 90% collagen in various forms. The extracellular matrices derived from various living tissues may differ in their overall structure and composition due to the unique role needed for each tissue.

The term "derive" or "derived" refers to a component obtained from any stated source by any useful method.

In an embodiment of the present disclosure, the EEM may be prepared from an esophageal tissue decellularized by treatment with trypsin/EDTA, Triton X-100, and sodium deoxycholate. Accordingly, the EEM may be decellularized.

In an embodiment of the present disclosure, a concentration of the EEN in the scaffold may be from 0.5 mg/ml to 10 mg/ml, specifically from 1 mg/ml to 7 mg/ml. The concentration of the EEM may be, for example, from 1 mg/ml to 7 mg/ml, from 1 mg/ml to 5 mg/ml, from 1 mg/ml to 3 mg/ml, from 3 mg/ml to 7 mg/ml, from 3 mg/ml to 5 mg/ml, or from 5 mg/ml to 7 mg/ml. In an example, the concentration of the EEM may be 1 mg/ml, 3 mg/ml, 5 mg/ml, or 7 mg/ml. The EEM contained with a concentration out of the above-described range may make it impossible to achieve the intended effect of the present disclosure.

The scaffold includes a three-dimensional hydrogel prepared based on the EEM obtained by decellularization, and can be effectively used for esophageal organoid culture.

The decellularized esophageal tissue contains actual tissue-specific extracellular matrix components and thus can provide the physical, mechanical and biochemical environment of the tissue, and is highly efficient in enhancing differentiation into esophageal tissue cells and tissue-specific functionality.

The term "organoid" refers to an ultraminiature body organ prepared in the form of an artificial organ by culturing cells derived from tissues or pluripotent stem cells in a 3D form.

The organoid is a three-dimensional tissue analog that contains organ-specific cells which originate from stem cells and self-organize (or self-pattern) in a similar manner to the in vivo condition. The organoid can be developed into a specific tissue by patterning a restricted element (for example, a growth factor).

The organoid can have the intrinsic physiological properties of the cells and can have an anatomical structure that mimics the original state of a cell mixture (including all remaining stem cells and the neighboring physiological niche as well as limited cell types). A three-dimensional culture method allows the organoid to be better arranged in terms of cell to cell functions and to have an organ-like form with functionality and a tissue-specific function.

Another aspect of the present disclosure provides a method of preparing a scaffold for culture and transplantation of an esophageal organoid, including: a process (1) of crushing an isolated esophageal tissue; and a process (2) of treating the crushed esophageal tissue with trypsin/EDTA, Triton X-100, and sodium deoxycholate for decellularization to prepare a decellularized EEM.

The process (1) is a process of crushing an isolated esophageal tissue, and the esophageal tissue may be isolated from a known animal. Examples of the animal may include cattle, pigs, monkeys, humans, etc. Also, in the present disclosure, the isolated esophageal tissue is crushed and then decellularized, which results in high efficiency in decellularization. The isolated esophageal tissue may be crushed by a known method.

The process (2) is a process of treating the crushed esophageal tissue with trypsin/EDTA, Triton X-100, and sodium deoxycholate for decellularization to prepare a decellularized EEM. According to the method of the present disclosure unlike a conventional decellularization method, it is possible to efficiently remove most of the cells without treatment with a deoxyribonuclease (DNase). The decellularization may be performed through a known process. For example, the decellularization may be performed by stirring the crushed esophageal tissue with trypsin/EDTA, Triton X-100, and sodium deoxycholate.

In an embodiment of the present disclosure, the method may further include, after the process (2), a process (3) of lyophilizing the decellularized EEM to prepare a lyophilized EEM.

The process (3) is a process of lyophilizing the decellularized EEM to prepare a lyophilized EEM. After drying, the lyophilized EEM may be exposed to electron beam, gamma radiation, ethylene oxide gas, or supercritical carbon dioxide for sterilization.

In an embodiment of the present disclosure, the method may further include, after the process (3), a process (4) of forming a scaffold for culture and transplantation of an esophageal organoid in the form of a hydrogel with the lyophilized EEM.

The process (4) is a process of forming a scaffold for culture and transplantation of an esophageal organoid in the form of a hydrogel with the lyophilized EEM. The process (4) may be performed through gelation. The decellularized EEM may be crosslinked to prepare a three-dimensional hydrogel-type scaffold, and the gelated scaffold can be used in various ways in the fields related to tests, screening and organoid culture.

The term "hydrogel" is a material in which a liquid that contains water as a dispersion medium is hardened through a sol-gel phase transition to lose fluidity and to form a porous structure. The hydrogel can be formed by causing a hydrophilic polymer that has a three-dimensional network structure and a microcrystalline structure to contain water and to be expanded.

The gelation may be performed at a temperature of 37°C for 30 minutes after dissolving the lyophilized EEM in an acidic solution with a protease such as pepsin or trypsin and adjusting a pH, specifically setting a neutral pH in an electrolyte state of 1X PBS buffer by using 10X PBS and 1 M NaOH.

Yet another aspect of the present disclosure provides a method of culturing an esophageal organoid in the above-described scaffold or a scaffold prepared by the above-described preparation method.

The conventional Matrigel-based culture system is an extract derived from animal cancer tissue, has a large difference between the batches, cannot mimic the actual esophageal environment, and exhibits insufficient efficiency in differentiation or development into an esophageal organoid. However, the scaffold can create an esophageal tissue-like environment and thus is suitable for esophageal organoid culture.

The culture refers to a process of maintaining and growing cells under suitable conditions, and the suitable conditions may refer to, for example, the temperature, nutrient availability, atmospheric CO₂ level and cell density at which the cells are maintained.

Appropriate culture conditions for maintaining, proliferating, expanding and differentiating different types of cells are known in the art and are documented. Suitable conditions for formation of the organoid may facilitate or allow cell differentiation and formation of a multicellular structure.

### MODE FOR CARRYING OUT THE INVENTION

In the present disclosure, a decellularized EEM scaffold was fabricated by effectively removing cells from an esophageal tissue through a series of chemical treatments, and a three-dimensional hydrogel was prepared from the decellularized EEM scaffold for use as an esophageal organoid culture matrix.

It was confirmed that in the decellularized EEM scaffold fabricated according to the present disclosure, most of the cells were removed, but various esophageal tissue-specific extracellular matrix components were well preserved. Therefore, the fabricated scaffold can be used as a matrix that can provide a tissue-specific microenvironment required for esophageal organoid culture.

It was confirmed that an esophageal organoid was actually formed in the fabricated decellularized EEM hydrogel scaffold, and the hydrogel scaffold was tested at various concentrations and an optimal concentration of the extracellular matrix for formation and development of an esophageal organoid was determined.

It was confirmed that an esophageal organoid cultured in a decellularized esophageal tissue-derived hydrogel scaffold was similar in morphology and growth pattern to an esophageal organoid cultured in a control group, Matrigel. Also, the presence of a basal layer and a suprabasal layer forming the esophagus was confirmed. Accordingly, it was verified that the decellularized scaffold according to the present disclosure has a potential as an esophageal organoid culture matrix that can replace Matrigel.

Esophageal organoids were cultured in decellularized esophagus, stomach, liver, heart, and skin tissue-derived hydrogel scaffolds, respectively, to compare the effects of tissue-specific extracellular matrix. In view of the organoid formation efficiency and the expression of two cell layers forming the esophageal tissue, the scaffold derived from the esophageal tissue was the most suitable for esophageal organoid culture compared to the scaffolds derived from the other tissues. Therefore, it was confirmed that the decellularized EEM scaffold can provide a tissue-specific microenvironment most suitable for the esophageal organoid.

The decellularized esophageal tissue-derived hydrogel scaffold was cold-stored for a long time and then used for culturing an esophageal organoid. As a result, it was confirmed that an esophageal organoid was formed at a similar level to that in a newly fabricated hydrogel scaffold and two types of esophageal tissue cell layer markers were expressed. This shows that the scaffold according to the present disclosure can be stored stably for a long term, suggesting a high possibility of commercialization of the decellularized esophageal tissue-derived hydrogel scaffold in the future.

The decellularized esophageal tissue-derived hydrogel scaffold was cryopreserved for a long time and then thawed to fabricate a hydrogel, and an esophageal organoid was cultured in the hydrogel. As a result, it was confirmed that an esophageal organoid was formed at a similar level to that in a newly fabricated hydrogel scaffold and two types of esophageal tissue cell layer markers were expressed. This shows that the scaffold according to the present disclosure can be used without any change in activity and performance even after cryopreservation for at least 3 months, suggesting a high possibility of commercialization of the decellularized esophageal tissue-derived hydrogel scaffold in the future.

It was confirmed that even when an esophageal organoid was cultured for a long time in the decellularized esophageal tissue-derived hydrogel scaffold with several times of subculture, an esophageal organoid was formed with a similar morphology and the two types of esophageal tissue cell layer markers were expressed at a level similar to or higher than that of the control group, Matrigel. This shows that an esophageal organoid can be cultured for a long time in the scaffold according to the present disclosure.

It was confirmed that when an esophageal organoid was transplanted into an esophagus injury mouse model using the decellularized esophageal tissue-derived hydrogel scaffold, the organoid was engrafted around the epithelium of the esophagus and effective in regenerating the damaged epithelium.

Hereinafter, Examples will be presented for understanding of the present disclosure. However, the following Examples are illustrative only for better understanding of the present disclosure but do not limit the present disclosure.

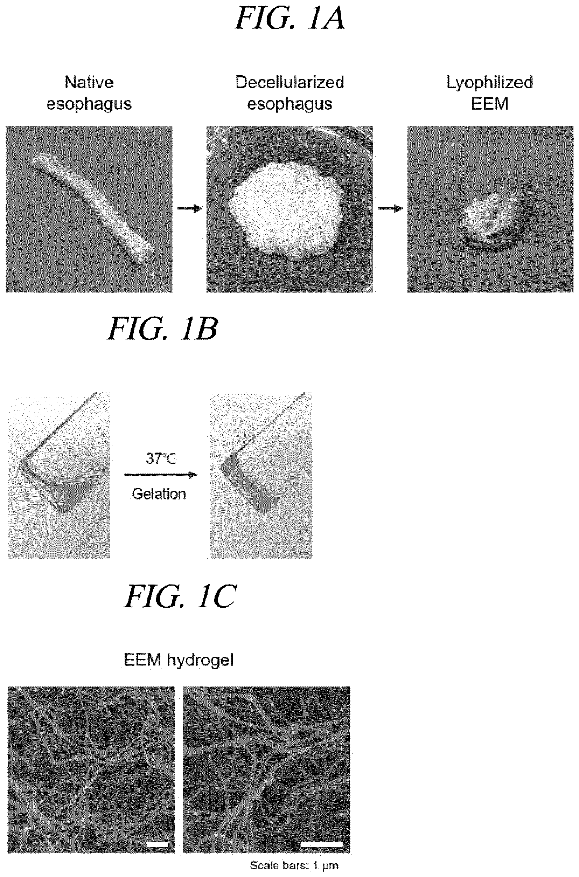

### Fabrication of decellularized esophageal tissue-derived scaffold and analysis of hydrogel microstructure (FIG. 1)

Cells were removed from a porcine esophageal tissue through decellularization to fabricate an EEM-based scaffold. In the decellularization process used in the present disclosure, the esophageal tissue was finely cut and then decellularized, and, thus, the cells were more effectively removed. Also, in the decellularization process used in the present disclosure unlike previously reported methods, most of the cells were efficiently removed without treatment with a DNase.

(A) A porcine esophageal tissue (Native esophagus) was effectively decellularized by a series of chemical treatments based on trypsin/EDTA, Triton X-100, and sodium deoxycholate and then crushed (Decellularized esophagus), followed by lyophilization to obtain an EEM (Lyophilized EEM).

(B) 10 mg of the lyophilized EEM was treated with 1 ml of a 4 mg/ml pepsin solution (a solution of 4 mg of pepsin powder derived from porcine gastric mucosa in 1 ml of 0.02 M HCl) and stirred at room temperature for 48 hours to carry out a solution process. After 10X PBS and NaOH were added into the prepared EEM solution to adjust the pH and electrolyte concentration suitable for cell culture, gelation was induced at 37°C for 30 minutes. Through this process, a solid hydrogel (right) was prepared and then used as a three-dimensional scaffold for organoid culture.

(C) A microstructure inside the three-dimensional EEM hydrogel was analyzed using a scanning electron microscope (SEM). As a result, the EEM hydrogel was observed to have a similar nanofibrous porous structure to a collagen hydrogel.

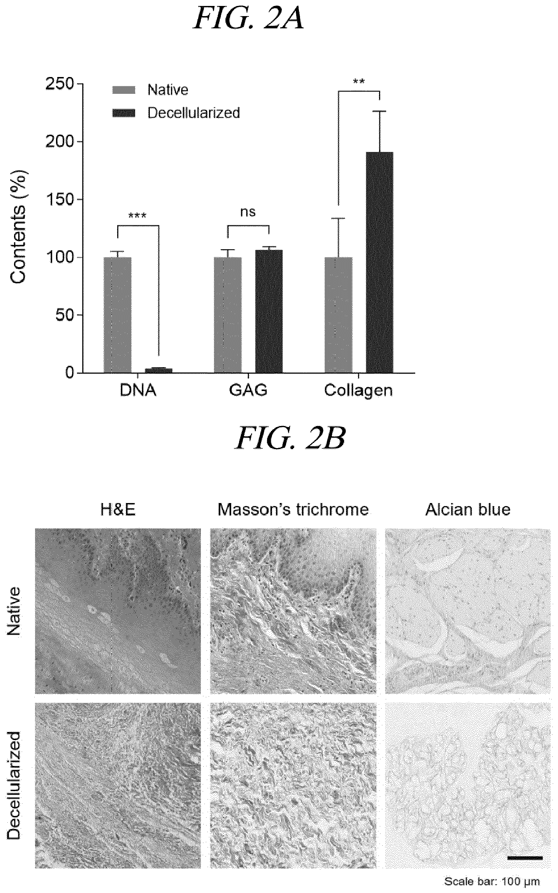

### Analysis of components of decellularized esophageal tissue-derived scaffold (FIG. 2)

(A) DNA, glycosaminoglycan (GAG) and collagen in porcine esophageal tissues before (Native) and after (Decellularized) decellularization were quantitatively analyzed and compared. As a result, it was confirmed that 96.37% of the cells were effectively removed through the decellularization process and the GAG and collagen components, which are the main components forming the extracellular matrix, were well maintained.

(B) Histological analysis was performed on porcine esophageal tissues before (Native) and after (Decellularized) decellularization. As a result, hematoxylin & eosin staining was performed to confirm that all of the cells were removed and the extracellular matrix component was preserved after the decellularization process. Also, Masson's trichrome staining was performed to confirm that the collagen components were well maintained even after the decellularization process. Further, Alcian blue staining was performed to confirm that the GAG components were well preserved.

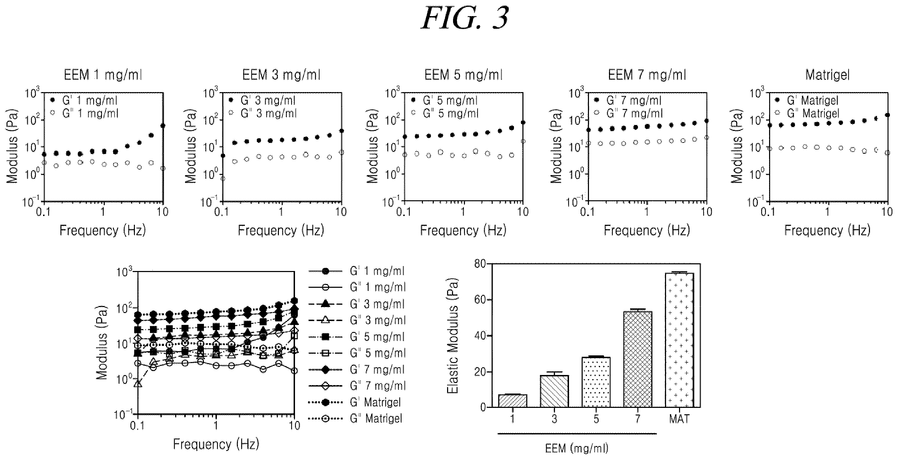

### Analysis of mechanical properties of decellularized esophageal tissue-derived hydrogel scaffold depending on concentration (FIG. 3)

Rheological analysis was performed to measure and compare the elastic modulus of the EEM hydrogel under four concentration conditions (1, 3, 5, 7 mg/ml) and Matrigel (MAT), which is a commercialized culture scaffold. It was confirmed that a stable polymer network was formed inside the EEM hydrogel by checking that a storage modulus G' value of the EEM hydrogel was consistently higher than a loss modulus G" value under all the concentration conditions as in Matrigel. Further, it was confirmed that as the concentration of EEM increased, the properties of the hydrogel increased and were generally lower than those of Matrigel.

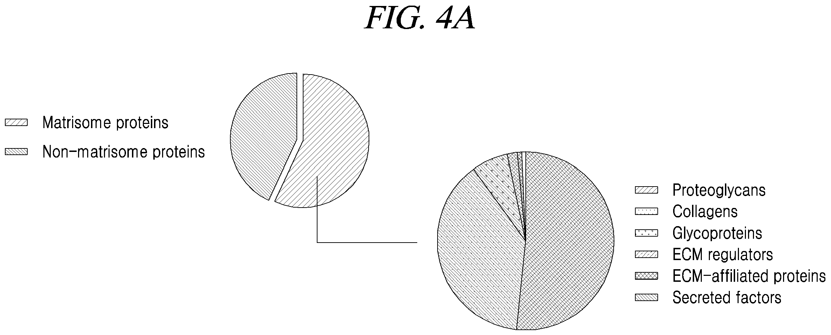

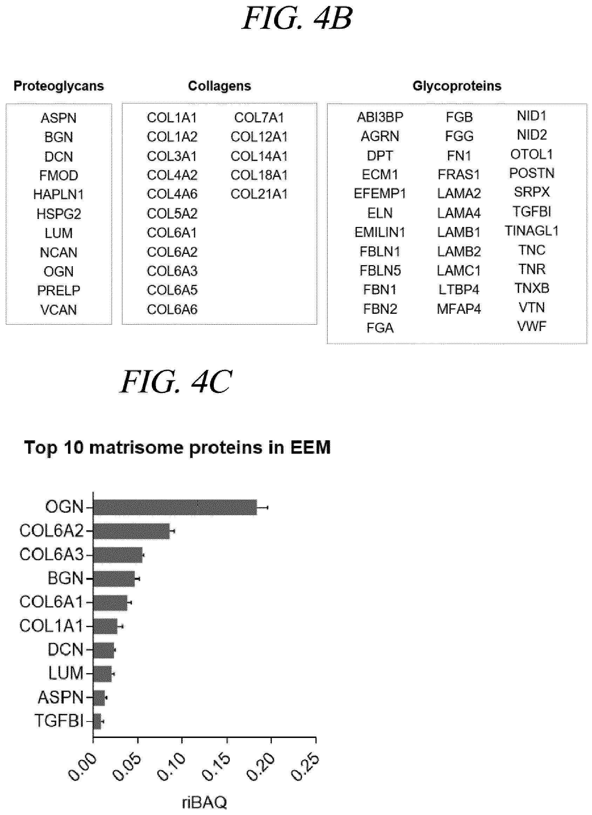

### Proteomics of decellularized esophageal tissue-derived scaffold (FIG. 4)

Proteomics of the EEM was performed using mass spectrometry.
(A) Among the proteins forming the EEM, extracellular matrix proteins (Matrisome proteins) accounted for 56.93%. In particular, the extracellular matrix proteins were composed mostly of proteoglycans, collagens, and glycoproteins.
(B) Each protein component belonging to proteoglycans, collagens, and glycoproteins as main extracellular matrix proteins forming the EEM was identified.
(C) Each protein component was quantified based on a relative intensity-based absolute quantification (riBAQ) value, and 10 protein components that accounted for the largest portion among the extracellular matrix proteins in the EEM were identified.

Accordingly, it is considered that the EEM scaffold composed mainly of the esophageal tissue extracellular matrix protein complex components can provide an extracellular matrix microenvironment suitable for esophageal organoid culture.

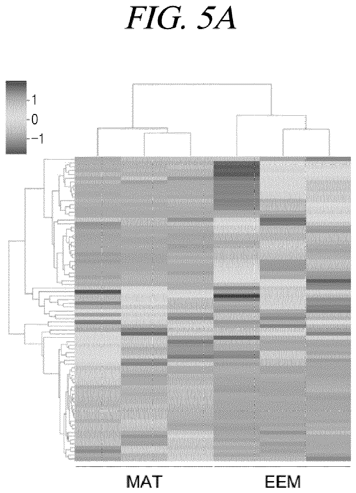

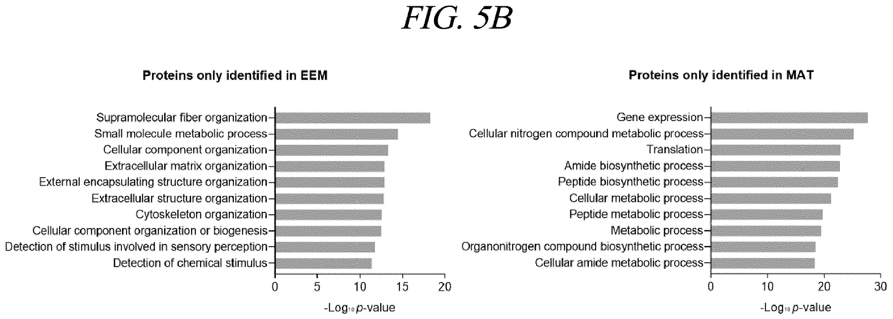

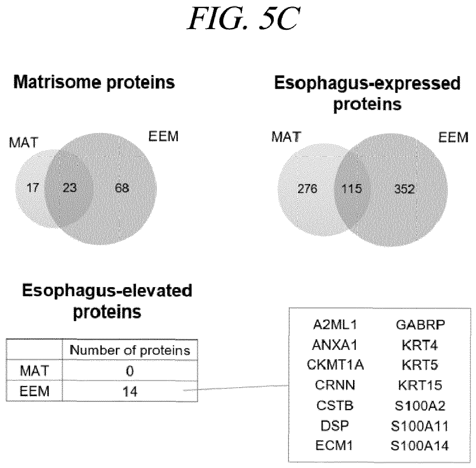

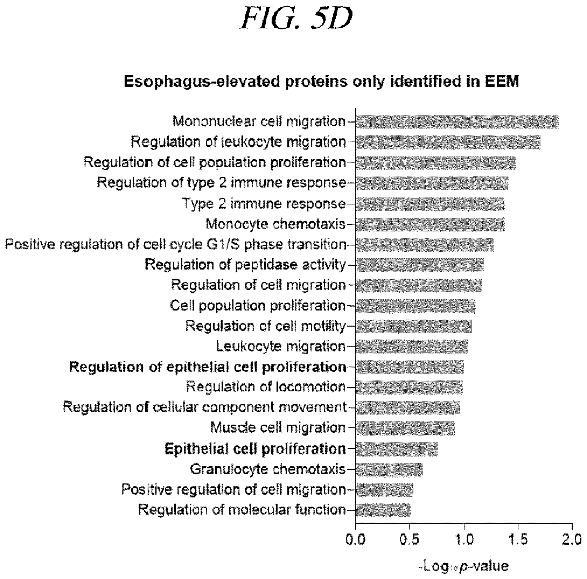

### Comparison between protein components of decellularized esophageal tissue-derived scaffold and protein components of Matrigel (FIG. 5)

(A) Differences in expression levels of protein components present in both the EEM and Matrigel were compared through a heat map. Accordingly, it was confirmed that there was a significant difference in composition of the protein components forming the two scaffolds.
(B) To analyze the protein components showing the difference between the EEM and Matrigel, gene ontology enrichment analysis was performed on each protein identified from only one of the two scaffolds, and the 10 most related biological processes were statistically identified according to p-values. It was confirmed that unlike Matrigel mainly related to cellular metabolic processes, the EEM was mainly related to extracellular matrix/structure organization and cytoskeleton organization.
(C) As a result of comparing the protein components forming the two scaffolds, more diverse types of extracellular matrix proteins (Matrisome proteins) were detected from EEM than from Matrigel, and more diverse types of esophagus-expressed proteins known to be expressed in the esophagus were also detected from the EEM. Also, esophagus-elevated proteins known to be expressed at least four times more in the esophagus than in other tissues were not detected at all from Matrigel and 14 types of esophagus-elevated proteins were detected from the EEM only.
(D) Gene ontology enrichment analysis was performed on the esophagus-elevated proteins that were not detected from Matrigel but detected from the EEM, and it was confirmed that these proteins are related to proliferation of epithelial cells.

Accordingly, it was confirmed that the EEM is a scaffold that can provide more diverse extracellular matrix components than Matrigel, which is a commercialized organoid culture scaffold, and in particular, it can create an esophagus-specific microenvironment. The esophagus-specific microenvironment is considered to be able to contribute to development of the epithelial cells forming the esophageal organoid.

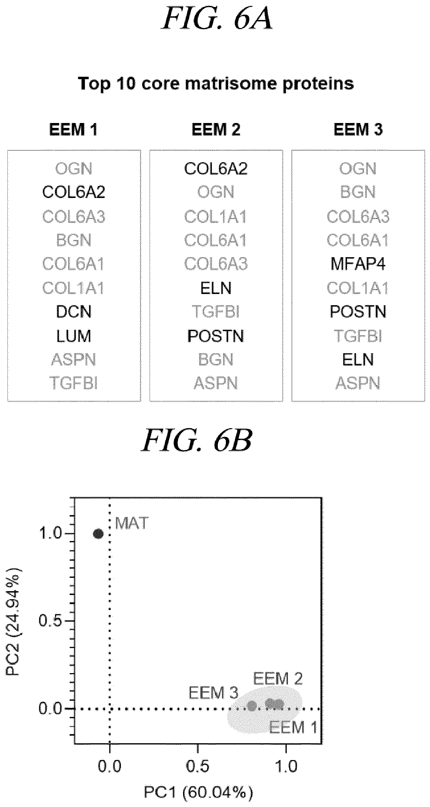

### Check of similarity between batches of decellularized esophageal tissue-derived scaffold (FIG. 6)

The similarity between batches was checked by comparing protein components forming EEMs prepared from different pigs.
(A) Each protein component was quantified based on the riBAQ value, and 10 components that accounted for the largest portion among the core matrisome proteins forming each EEM in three batches were compared. It was confirmed that the batches showed a fairly similar pattern (components common to all the three batches are indicated in red).
(B) Principal component analysis (PCA) was performed on the extracellular matrix proteins forming the EEM and Matrigel in the three batches to check the similarity. It was confirmed that the EEM batches showed a higher similarity in expression of extracellular matrix proteins than the Matrigel batches.

In view of the similarity in protein components between the EEM batches, it is considered that an EEM microenvironment can be repeatedly implemented even with EEMs from different batches.

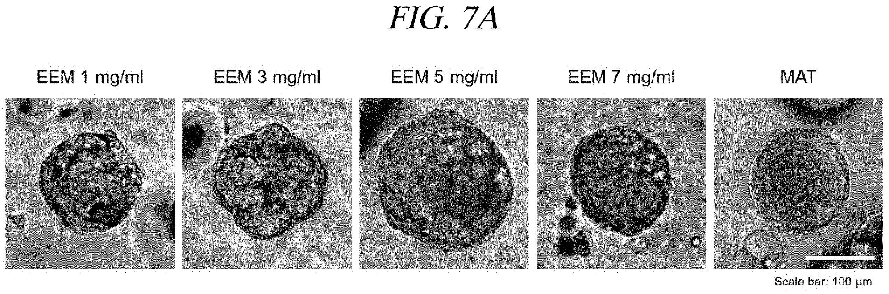

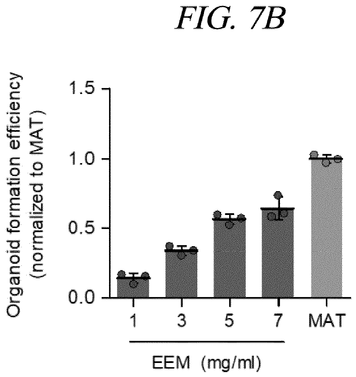

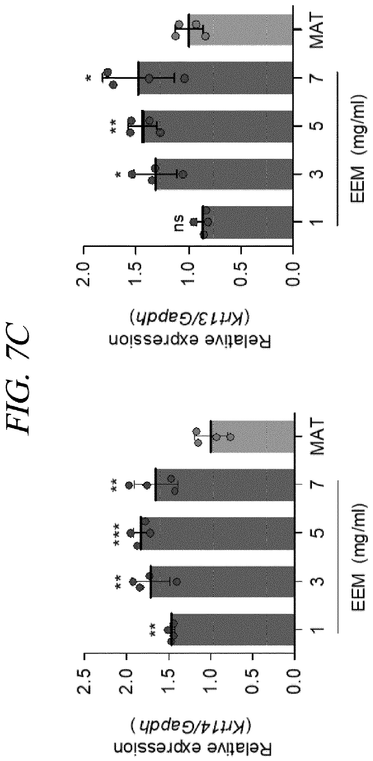

### Determination of optimal concentration of decellularized esophageal tissue-derived hydrogel scaffold for esophageal organoid culture (FIG. 7)

After the muscle layer of a mouse esophageal tissue was removed, the cells were extracted through an enzyme treatment and then cultured in an appropriate three-dimensional culture scaffold to form an esophageal organoid. In order to select an optimal concentration of the EEM hydrogel scaffold according to the present disclosure for esophageal organoid culture, the morphology, formation efficiency, and gene expression of esophageal organoids cultured in the EEM hydrogels at respective concentrations (1, 3, 5, 7 mg/ml) were compared to those of an esophageal organoid cultured in Matrigel, which is a commercialized culture scaffold.
(A) Esophageal organoids were formed in all the EEM hydrogels under the four concentration conditions, and it was confirmed that they have a similar morphology to the esophageal organoid cultured in Matrigel.
(B) The organoid formation efficiency for each EEM hydrogel concentration was compared to that of Matrigel. Overall, the EEM hydrogels showed a lower formation efficiency than Matrigel, but showed a higher formation efficiency at 5 mg/ml and 7 mg/ml than at the other concentrations.
(C) The mRNA expression levels for specific genes of the esophageal organoids cultured in the EEM hydrogels under the four concentration conditions and Matrigel were compared by quantitative PCR. The expression of *Krtl4,* a gene expressed in the basal layer of the esophagus, was increased in the EEM hydrogels at all the concentration conditions (left), and *Krt13,* a gene expressed in the suprabasal layer, was increased in the EEM hydrogels at 3, 5, and 7 mg/ml (right). It was confirmed that the expression of the both genes expressed in the basal layer and the suprabasal layer, respectively, was generally increased in the EEM hydrogels. In particular, the degree of increase in expression of an esophageal tissue marker was the most significant in the 5 mg/ml EEM group compared to the Matrigel group. Accordingly, the optimal concentration of the EEM for esophageal organoid culture was set to 5 mg/ml and then applied to culture with an EEM hydrogel.

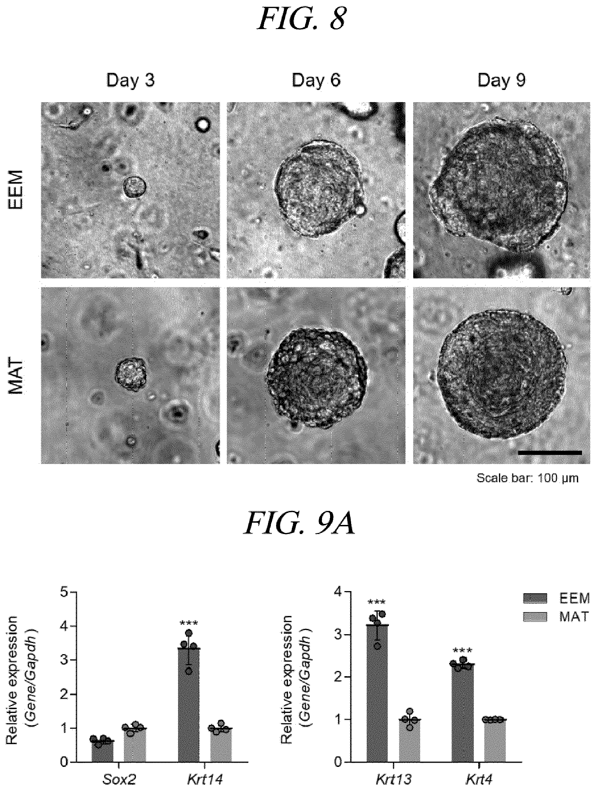

### Check of growth pattern of esophageal organoid cultured in decellularized esophageal tissue-derived hydrogel scaffold (FIG. 8)

The growth patterns of the esophageal organoids cultured in the 5 mg/ml EEM hydrogel and Matrigel were checked on the 3rd, 6th, and 9th days (Days 3, 6, and 9) of culture. It was confirmed that the esophageal organoid cultured in the EEM hydrogel increased in size at a similar growth rate to that in Matrigel.

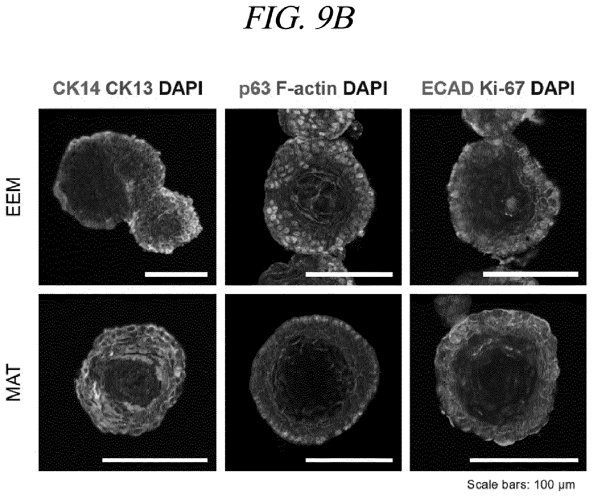

### Analysis of gene and protein expression in esophageal organoid cultured in decellularized esophageal tissue-derived hydrogel scaffold (FIG. 9)

(A) The gene expression of the esophageal organoids cultured in the 5 mg/ml EEM hydrogel and Matrigel was compared by quantitative PCR. When the expression of *Sox2* and *Krtl4,* genes expressed in the basal layer, was compared, the expression of *Sox2* was not significantly different, but the expression of *Krtl4* was significantly increased in the EEM hydrogel group (left). Also, when the expression of *Krt13* and *Krt4,* genes expressed in the suprabasal layer, was compared, the expression of the both genes was significantly increased in the EEM hydrogel group compared to the Matrigel group (right).
(B) The protein expression of the esophageal organoids in the 5 mg/ml EEM hydrogel and Matrigel was compared by immunofluorescence staining. It was confirmed that cytokeratin 14 (CK14) and p63, proteins expressed in the basal layer, were expressed with similar morphologies on the outermost part of the organoid in the both groups, and cytokeratin 13 (CK13), a protein expressed in the suprabasal layer, was also expressed at similar levels in the both groups. Also, it was confirmed from the expression patterns of F-actin, a major protein forming the cytoskeleton, and E-cadherin (ECAD), a protein expressed at the junction between epithelial cells, that a spherical esophageal organoid consisting of adjacent epithelial cells was formed in the EEM hydrogel as in Matrigel. Further, it was confirmed that Ki-67, a protein related to cell proliferation, was expressed at similar levels.

Accordingly, it was confirmed that the EEM hydrogel is a scaffold capable of forming an esophageal organoid at a similar level to that in Matrigel, which is a conventionally commercialized organoid culture scaffold.

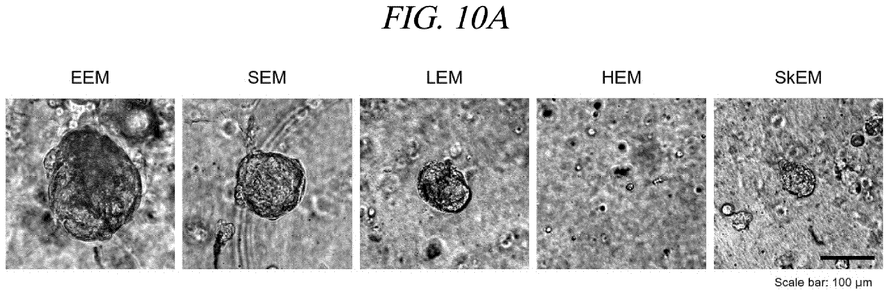

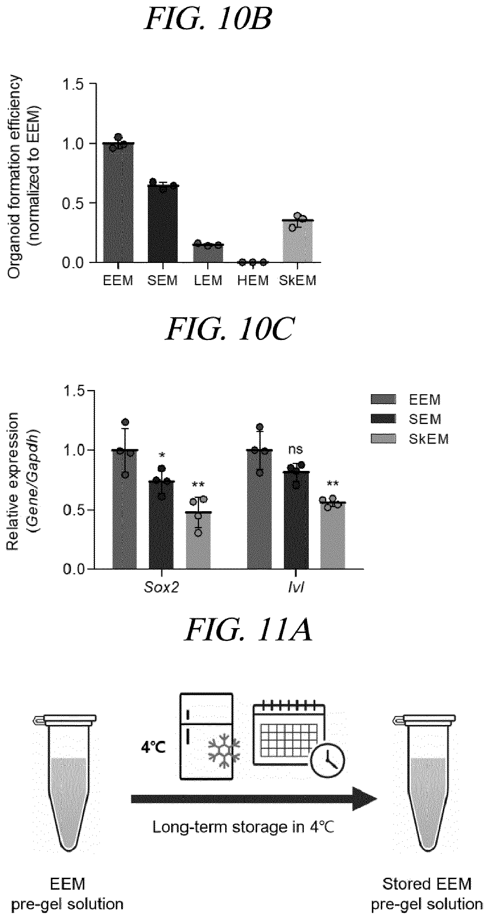

### Check of tissue-specific effect of decellularized esophageal tissue-derived scaffold for esophageal organoid culture (FIG. 10)

To confirm that the EEM hydrogel scaffold derived from the decellularized esophageal tissue provides a tissue-specific microenvironment for esophageal organoid culture, esophageal organoids were cultured in hydrogel scaffolds derived from other decellularized tissues and the organoid formation and gene expression were compared to those of the EEM hydrogel scaffold. Decellularized stomach (SEM), liver (LEM), heart (HEM), and skin (SkEM) tissues were used for the other scaffolds.
(A) It was confirmed that esophageal organoids were formed in the scaffolds derived from the decellularized stomach, liver, and skin tissues, but not in the scaffold derived from the decellularized heart tissue.
(B) As a result of comparing the organoid formation efficiency in each scaffold, it was confirmed that the formation efficiency was greatly decreased in all the scaffolds derived from the other tissues.
(C) The gene expression of the esophageal organoids cultured in the scaffolds derived from the stomach and skin tissues, except for the LEM and HEM groups where mRNA cannot be extracted due to very low esophageal organoid formation efficiency, was compared to that of the organoid cultured in the esophageal-derived scaffold by quantitative PCR. The expression of *Sox2,* a gene expressed in the basal layer, was significantly decreased in the esophageal organoids cultured in the scaffolds derived from the stomach and skin tissues. In particular, the expression of *Ivl,* a gene expressed in the suprabasal layer, was also significantly decreased in the esophageal organoid cultured in the scaffold derived from the skin tissue.

Through this test, it was confirmed that the formation and development of an esophageal organoid was most suitable in the EEM hydrogel scaffold compared to the hydrogel scaffolds derived from tissues other than the esophagus. As a result, it was confirmed that the EEM hydrogel scaffold can provide an esophageal tissue-specific microenvironment.

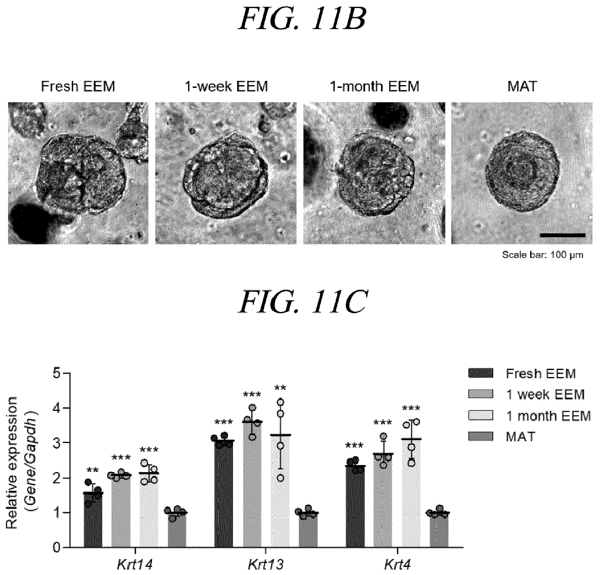

### Verification on possibility of long-term cold storage of decellularized esophageal tissue-derived hydrogel scaffold (FIG. 11)

(A) A decellularized esophageal tissue-derived hydrogel pre-gel solution was cold-stored at 4°C for a long time and then used to prepare hydrogels. Esophageal organoids were cultured in the prepared hydrogels, and the results of culture were compared to verify the possibility of long-term cold storage of an EEM scaffold.
(B) It was confirmed that esophageal organoids were formed in an EEM hydrogel prepared immediately before culture, and EEM hydrogels prepared with the pre-gel solutions stored at 4°C for 1 week and 1 month, respectively, with similar morphologies to that in Matrigel.
(C) The gene expression of the esophageal organoids cultured in the EEM hydrogel prepared immediately before culture, the EEM hydrogels prepared with the pre-gel solutions stored at 4°C for 1 week and 1 month, respectively, and Matrigel was compared by quantitative PCR. The expression of *Krtl4,* a gene expressed in the basal layer, and the expression of *Krt13* and *Krt4,* genes expressed in the suprabasal layer, were significantly increased in the EEM hydrogel group compared to the Matrigel group.

Through this test, it was confirmed that the EEM hydrogel scaffold did not show any change in performance as an esophageal organoid culture scaffold even after it was cold-stored as a pre-gel solution for 1 month. Such excellent storage properties serve as an advantage for the commercialization of the developed scaffold.

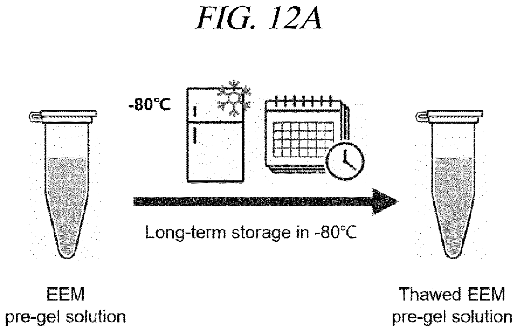

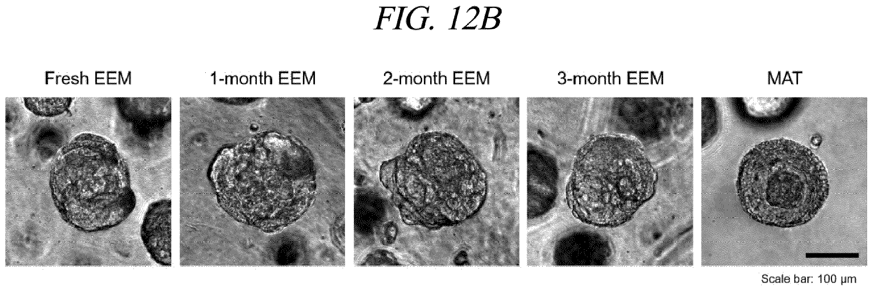

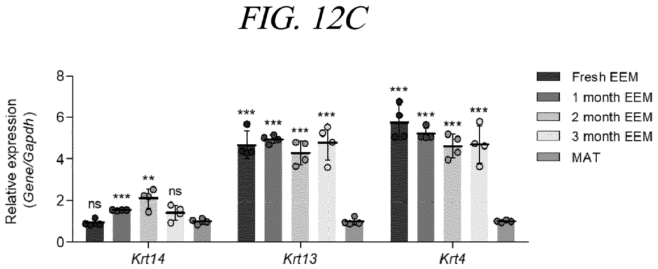

### Verification on possibility of long-term cryopreservation of decellularized esophageal tissue-derived hydrogel scaffold (FIG. 12)

(A) A decellularized esophageal tissue-derived hydrogel pre-gel solution was cryopreserved at -80°C for a long time and then thawed and used to prepare hydrogels. Esophageal organoids were cultured in the prepared hydrogels, and the results of culture were compared to verify the possibility of long-term cryopreservation of an EEM scaffold.
(B) It was confirmed that esophageal organoids were well formed in an EEM hydrogel prepared immediately before culture with a fresh EEM solution, and EEM hydrogels prepared with the pre-gel solutions stored at -80°C for 1 month, 2 months, and 3 months, respectively, with similar morphologies to that in Matrigel.
(C) The gene expression of the esophageal organoids cultured in the EEM hydrogel prepared immediately before culture, the EEM hydrogels prepared with the pre-gel solutions stored at -80°C for 1 month, 2 months, and 3 months, respectively, and Matrigel was compared by quantitative PCR. The expression of *Krtl4,* a gene expressed in the basal layer, and the expression of *Krt13* and *Krt4,* genes expressed in the suprabasal layer, remained similar or significantly increased in the EEM hydrogel group compared to the Matrigel group. The same patterns were observed from the organoid groups cultured in the cryopreserved hydrogels.

Through this test, it was confirmed that the EEM hydrogel did not show any change in activity and performance as an esophageal organoid culture scaffold even after it was cryopreserved as a pre-gel solution for at least 3 months. Such excellent storage properties serve as an advantage for the commercialization of the developed scaffold.

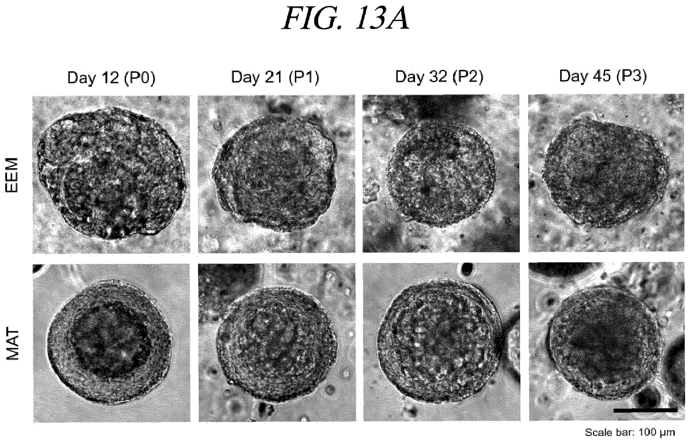

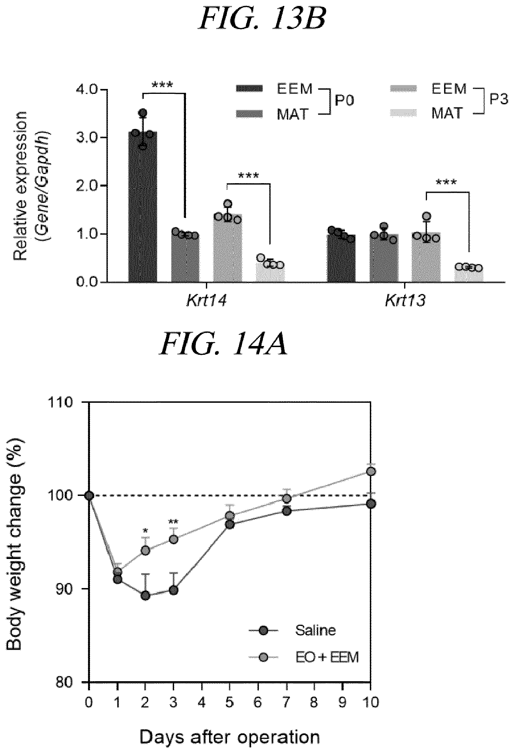

### Verification on long-term culture of esophageal organoid in decellularized esophageal tissue-derived hydrogel scaffold (FIG. 13)

(A) The possibility of subculture was verified by culturing an esophageal organoid in an EEM hydrogel for a total of 45 days with three times of subculture. Even when cultured for 45 days, the esophageal organoid was formed with a similar morphology to that in Matrigel.
(B) The gene expression of esophageal organoids cultured in the EEM hydrogel and Matrigel for 12 days (P0) and 45 days (P3) was compared by quantitative PCR. As a result, when the expression of *Krtl4,* a gene expressed in the basal layer, and *Krt13,* a gene expressed in the suprabasal layer, was compared, the expression of the both genes remained similar or significantly increased in the EEM hydrogel group compared to the Matrigel group. The same results were also observed when cultured for 45 days with subculture.

Accordingly, it was confirmed that the esophageal organoid can be cultured for a long time in the EEM hydrogel. Also, it was confirmed that even after several times of subculture, the EEM hydrogel showed a similar or improved esophagus-specific gene expression pattern compared to Matrigel.

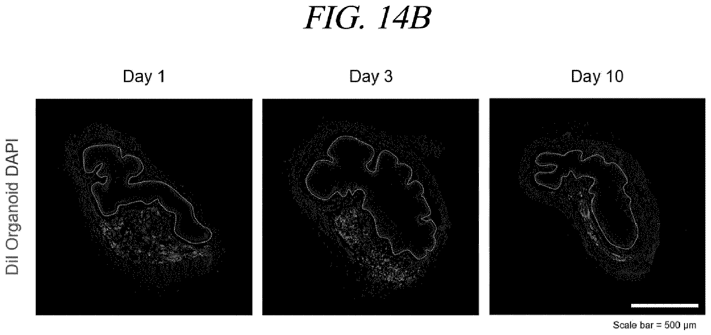

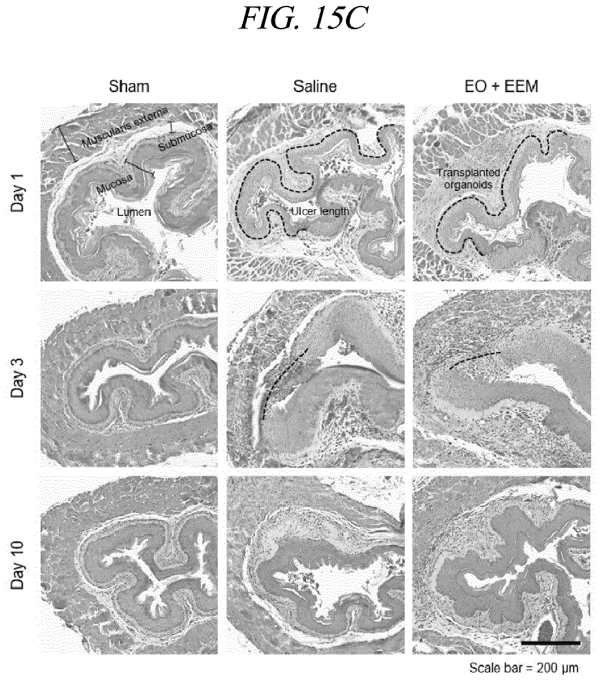

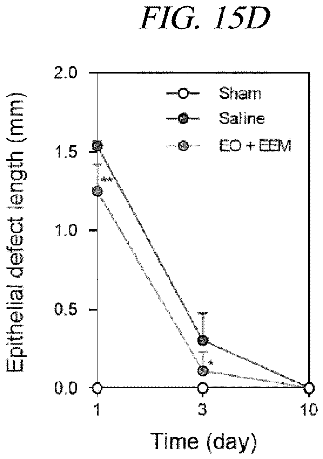

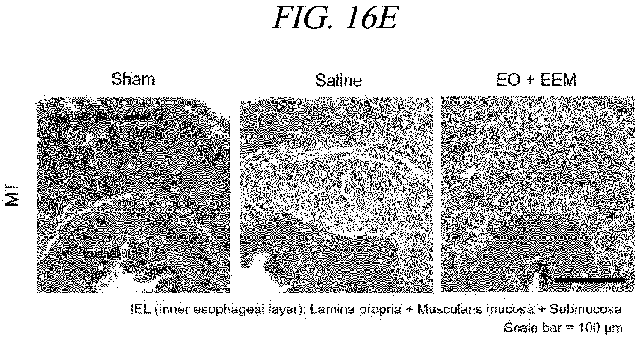

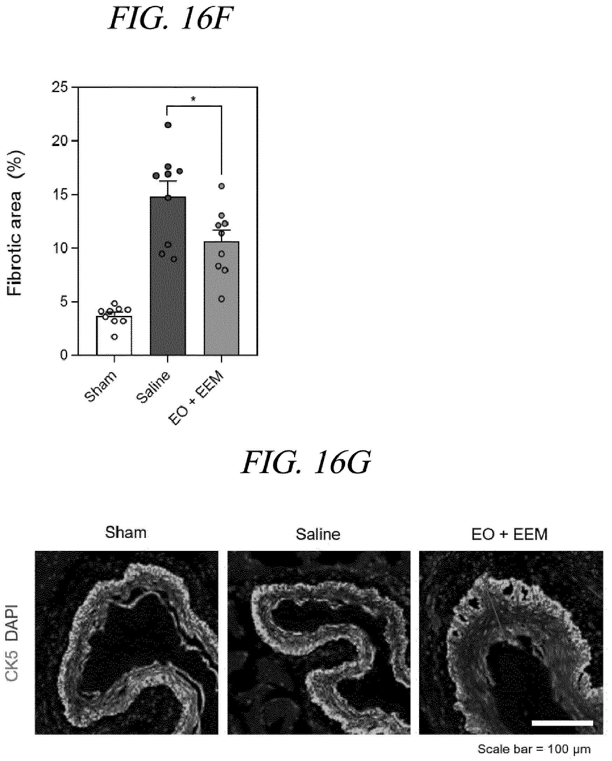

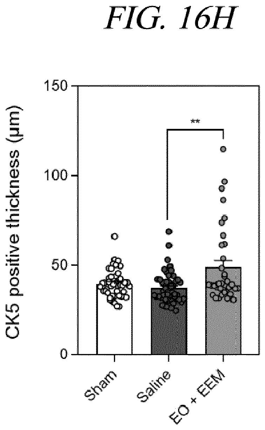

### Verification on in vivo transplantation and regeneration effects of esophageal organoid using decellularized esophageal tissue-derived hydrogel (FIG. 14 to FIG. 16)

An esophagus injury mouse model was prepared by inducing ulceration through treatment of the outer wall of the esophagus with 50% acetic acid for 30 seconds using a capillary tube. Immediately after the injury, an esophageal organoid cultured in an EEM was submucosally injected into the esophagus injury mouse model by using 5 mg/ml EEM hydrogel. To check engraftment of the organoid, the esophageal was labeled with Dil. In this test, about 1000 esophageal organoids per mouse were transplanted using 20 µl of an EEM pre-gel solution. Also, a no injury group (Sham), a group injected with saline after the injury (Saline), and a group in which the esophageal organoids were transplanted using EEM hydrogels after the injury (Esophageal organoid + EEM; EO + EEM) were compared to check the regeneration effect.

(A) As a result of measuring the body weight of the mice after the injury, both the Saline group and the EO + EEM group showed a decrease in body weight in the beginning and recovered with time. In particular, on days 2 and 3, the EO + EEM group showed a significant difference in the degree of weight recovery compared to the Saline group.

(B) The tissues of the EO + EEM group were collected on days 1, 3, and 10 after transplantation to check the engraftment of the Dil-labeled esophageal organoids. It was confirmed that the organoids were located under the esophageal epithelium. In particular, the organoids were stably engrafted and remained at the same location till day 10.

(C, D) The tissues were collected on days 1, 3, and 10 after transplantation, and H&E staining was performed to observe morphological changes of the esophageal tissue. Damage to the mucosa epithelium was observed in the Saline group and the EO + EEM group compared to the Sham group, and the damage area was decreased in length (as indicated by the black dotted line) and reepithelialized with time. The length of the epithelial damage area observed through H&E staining was measured and compared. On days 1 and 3, the degree of recovery of the damage area in the EO + EEM group was significantly different from that in the Saline group.

(E, F) Masson's trichrome staining was performed to compare the degree of fibrosis in the tissues collected on day 10 after transplantation based on collagen accumulated in the inner esophageal layer (IEL) between the esophageal epithelium and the muscularis externa. Fibrosis was induced after the injury in the Saline group and the EO + EEM group compared to the Sham group, but the degree of fibrosis decreased in the EO + EEM group.

(G, H) The tissues collected on day 10 after transplantation were immunostained with Cytokeratin 5 (CK5) expressed in the basal layer of the esophagus and then compared. The thickness of the layer where CK5 is expressed was significantly increased in the EO + EEM group compared to that in the Saline group. Based on this result, it was confirmed that the regeneration of the damaged esophageal epithelium was promoted when the esophageal organoid was transplanted using the EEM hydrogel.

Accordingly, it was confirmed that the EEM hydrogel enables transplantation and engraftment of an esophageal organoid and is effective in regenerating damaged esophageal epithelium and suppressing fibrosis.

The above description of the present disclosure is provided for the purpose of illustration, and it would be understood by a person with ordinary skill in the art that various changes and modifications may be made without changing technical conception and essential features of the present disclosure. Thus, it is clear that the above-described examples are illustrative in all aspects and do not limit the present disclosure.

## Claims

1. A scaffold for culture and transplantation of an esophageal organoid, containing an esophagus extracellular matrix (EEM).

2. The scaffold of Claim 1,
wherein the esophagus extracellular matrix is decellularized by treating an esophageal tissue with trypsin/EDTA, Triton X-100, and sodium deoxycholate.

3. The scaffold of Claim 1,
wherein a concentration of the esophagus extracellular matrix in the scaffold is from 0.5 mg/ml to 10 mg/ml.

4. A method of preparing a scaffold for culture and transplantation of an esophageal organoid, comprising:
a process (1) of crushing an isolated esophageal tissue; and
a process (2) of treating the crushed esophageal tissue with trypsin/EDTA, Triton X-100, and sodium deoxycholate for decellularization to prepare a decellularized esophagus extracellular matrix.

5. The method of preparing a scaffold for culture and transplantation of an esophageal organoid of Claim 4, further comprising:
after the process (2), a process (3) of lyophilizing the decellularized esophagus extracellular matrix (EEM) to prepare a lyophilized esophagus extracellular matrix.

6. The method of preparing a scaffold for culture and transplantation of an esophageal organoid of Claim 5, further comprising:
after the process (3), a process (4) of forming a scaffold for culture and transplantation of an esophageal organoid in the form of a hydrogel with the lyophilized esophagus extracellular matrix.

7. The method of preparing a scaffold for culture and transplantation of an esophageal organoid of Claim 6,
wherein in the process (4), the lyophilized esophagus extracellular matrix is dissolved in a pepsin solution and a pH of the solution is adjusted to form the hydrogel.

8. A method of culturing an esophageal organoid in the scaffold of Claim 1 or a scaffold prepared by the preparation method of Claim 4.
